# EUROPEAN PATENT SPECIFICATION

(11) **EP 3 378 500 B1**
(45) Date of publication and mention of the grant of the patent: **29.06.2022**
(21) Application number: 18157787.5
(22) Date of filing: 31.03.2016
(51) Int. Cl.: A61L 2/20, B01B 1/00

(54) **MACHINE FOR STERILISING CONTAINERS**
MASCHINE ZUM STERILISIEREN VON BEHÄLTERN
MACHINE DE STÉRILISATION DE RÉCIPIENTS

(30) Priority: 24.06.2015 IT UB20151618
(43) Date of publication of application: 26.09.2018
(62) Divisional of application: 16163179.1
(73) Proprietor: Gea Procomac S.p.A., 43038 Sala Baganza (PR) (IT)
(72) Inventor: ABELLI, Paolo, 43123 PARMA (IT)
(74) Representative: Dondi, Silvia

(56) References cited:
- EP-A2- 0 321 908
- EP-A2- 0 956 054
- WO-A2-2009/013226
- US-A- 5 173 259
- US-B2- 6 919 043

## Description

The present invention relates to a machine for sterilising containers.

The reference sector is the bottling of so-called "sensitive" food products, i.e. products that are particularly sensitive to bacteriological contamination and oxidation, such as, for example, isotonic drinks, juices, nectars, soft drinks, teas, milk-based drinks, coffee-based drinks, etc., for which it is fundamental to prevent any microbiological contamination throughout the packaging stages.

In this context, the attention is focused on the decontamination of the primary packaging with which the product comes into contact, whether it is a bottle (or its parison), a pouch or flexible container or a cardboard container ("brick").

In particular, the present invention relates to the aseptic packaging of drinks in bottles made of thermoplastic material (preferably PET) of which the parison is sterilised. The sterile parison is then blown under sterile conditions, so as to obtain a sterile bottle ready to be filled directly, without intermediate sterilisation or rinsing steps.

Decontamination techniques of the prior art for containers envisage the use of chemical agents (e.g. aqueous solutions containing peracetic acid or hydrogen peroxide), or the application of radiation or the use of heat.

Chemical agents commonly used are peracetic acid and hydrogen peroxide. Throughout the present description and appended claims, the term "hydrogen peroxide" refers to an aqueous solution of hydrogen peroxide, preferably 35%.

The application of hydrogen peroxide generally takes place according to two methods.

A first chemical sterilisation method for sterilising the walls of a container envisages the following steps:
- vaporising an aqueous solution containing hydrogen peroxide into the container;
- making the hydrogen peroxide vapours condense on the inside walls of the container (bringing the walls themselves to a temperature less than or equal to the condensation temperature of the peroxide vapours);

- making the condensate evaporate, e.g. with hot air, so as to "activate" the hydrogen peroxide previously condensed;
- drying the inside walls of the container.

A second chemical sterilisation method envisages keeping the hydrogen peroxide constantly in the vapour state. The hydrogen peroxide is vaporised and the vapours are sent onto the surface to be sterilised. Having such surface at a temperature greater than the hydrogen peroxide vapour temperature means condensation does not occur throughout the entire sterilisation period. In this case, given the temperature of the vapours, it is not necessary to activate the hydrogen peroxide.

Both methods envisage a step in which an aqueous solution of hydrogen peroxide is brought from the liquid phase to the gaseous phase, known as VHP (Vapour Hydrogen Peroxide).

To generate VHP it is known that the liquid hydrogen peroxide can be dripped onto a hot plate (for example heated with electrical resistances) having a temperature sufficient to make the hydrogen peroxide evaporate. Within the plate vaporiser the supply of another fluid is envisaged, typically air, which acts as a support, i.e. it has the task of transporting the hydrogen peroxide vapours towards the surface to be sterilised. An example of this type of vaporiser is described in WO 2009/013226.

US5173259A discloses a vaporizer to produce an air / VHP sterilant for treating a flat, foldable packaging foil, the sterilant thereby attaining a temperature relatively higher than the dew point to avoid potential condensation.

Another apparatus, known for example by document EP1135213, envisages the use of a nebuliser nozzle to atomise the hydrogen peroxide into an air flow, which again has a support function. The mixture of air and nebulised hydrogen peroxide passes through a heat exchanger so as to change the state of the hydrogen peroxide from liquid to vapour.

Finally, document WO98/34649 describes a vaporisation device split into two parts: the liquid steriliser is vaporised into the first part of the device and subsequently the sterilising vapours undergo a second heating, still within the same vaporisation device.

In all the known examples, the vaporisation apparatus must be arranged as near as possible to the zone in which the decontamination takes place, ideally close to the steriliser. In fact, it is fundamental for the hydrogen peroxide to completely evaporate and remain in the gaseous phase until it is time for sterilisation. The complete evaporation of the hydrogen peroxide allows the process to be kept under control over time so as to control the concentration of the steriliser on the surface to be treated.

In fact, in the process it is necessary to provide the correct quantity of liquid hydrogen peroxide to be vaporised per hour, which depends both on the desired concentration in the gaseous phase within the support fluid (connected with the required sterilisation performance and depending on the type of product filled) and on the flow rate of the support fluid (connected with how many containers need to be sterilised per hour, therefore with the size of the sterilising machine).

If at some points of the system, between the vaporisation and the sterilisation, the hydrogen peroxide vapours condensed, even in small quantities, the concentration value of the steriliser would be reduced and control of the process would be lost, reaching conditions in which the decontamination may not be effective.

In the process control, the temperature of the hydrogen peroxide vapours must be kept above the minimum value to guarantee sterilisation effectiveness. This aspect is particularly significant in the production of low-acid drinks, which require higher performance sterilisation for the packaging than other drinks.

A fundamental requirement in sterilising packaging intended to contain drinks is that the sterilising effect is greater than or equal to the value established and that it remains constant over time.

To obtain this condition it is important, as mentioned above, that condensate does not form and that the temperature of the vapours is above the threshold value. The two phenomena are related: by managing to keep the system above the dew point the formation of "cold" areas where the vapours could condense is less likely.

By keeping the vaporisation system as close as possible to the point in which the sterilisation takes place, the temperature of the vapours would be more easily kept above the minimum values and the probability of forming condensate would be reduced.

However, such positioning is not possible when the quantity per hour of liquid hydrogen peroxide to be vaporised becomes significant (e.g. over 6 kg/h) and therefore the dimensions of the vaporisation apparatus become such that they are not compatible with the available space on board or near the sterilising machine. In this case, the connection piping between the vaporiser and the steriliser, although insulated or even made in some sections with electrically heated pipes, is a heat dissipation element such that the temperature of the vapours at the sterilisation nozzles (i.e. in the point at which they come into contact with the packaging) may not exceed the minimum value for being able to guarantee the required sterilisation performance.

In these cases, it is useless or even negative to increase the temperature of the vaporisation apparatus. In fact, increasing the temperature of the vapours in contact with the packaging is not proportional to the increase in vaporisation temperature and increasing the vaporisation temperature generates in the vaporiser degradation of the hydrogen peroxide and calefaction, which reduce the vaporisation efficiency.

The situation becomes even more critical when the machine restarts, for example, after an interruption due to a prolonged stop of the line. In fact, after a prolonged stop, before starting the vaporisation of the hydrogen peroxide, the system must be brought to a higher temperature than the dew point to prevent the formation of condensate and to guarantee that the temperature of the vapours exceeds the minimum value. Therefore, a certain period of time is required, which represents downtime with no production.

In this context, the technical task underpinning the present invention is to provide a machine for sterilising containers which obviates the drawbacks of the prior art as cited above.

In particular, an object of the present invention is to provide a machine for sterilising containers that ensures the desired degree of sterility is obtained and maintained over time, without having to increase the structural complexity.

The stated technical task and specified objects are achieved by a machine for sterilising containers according to claim 1, which defines the invention. Particular embodiments of that invention are comprised in the dependent claims.

Additional features and advantages of the present invention will become more apparent from the approximate and thus non-limiting description of a preferred but not exclusive embodiment of a machine for sterilising containers.

This description will be given below with reference to the attached drawings, provided solely for illustrative and therefore non-limiting purposes, in which:
- figure 1 illustrates a a schematic plan view of a machine for sterilising containers, according to the present invention;
- figure 2 illustrates a part of the machine for sterilising containers, in accordance with a first embodiment of the invention;

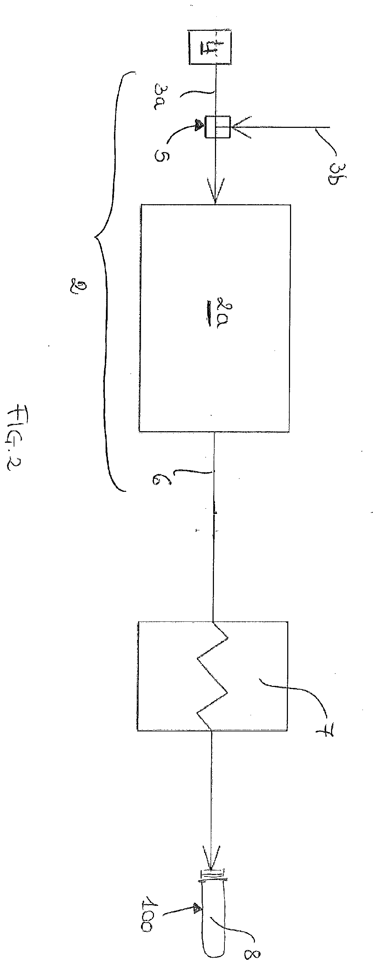

- figure 2 illustrates a a part of the machine for sterilising containers, in accordance with a second embodiment of the invention.

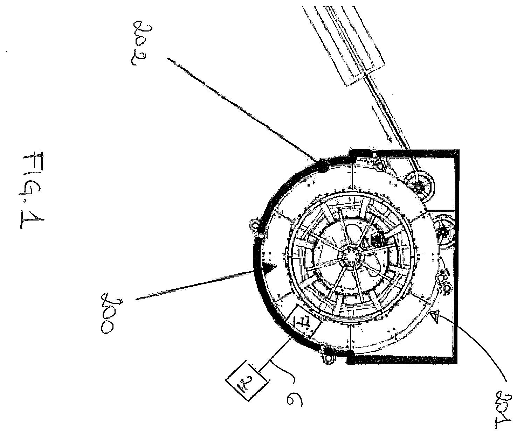

With reference to figure 1, 200 indicates a machine for sterilising containers 100, hereinafter indicated for short as "machine" 200.

In particular, the container 100 is a parison or a bottle made of polyethylene (known as PET, which stands for polyethylene terephthalate) or high-density polyethylene (known by the acronym HDPE).

It is also possible to use the same machine 200 for sterilising a capsule or a cap.

Alternatively, the container 100 is a pouch or flexible container, or a cardboard container.

The machine 200 comprises a rotary carousel 201 provided with a plurality of treatment stations for treating the containers 100. In each treatment station a dispensing nozzle (not illustrated) is arranged.

The machine 200 comprises an isolator 202 configured to separate the rotary carousel 201 from the external environment.

An example of a system comprising isolators suitable for application in the machine 200 is described and illustrated in document EP2279850

The machine 200 further comprises a vaporisation device 2 configured to generate a mixture comprising air, water vapour and an evaporated sterilising agent.

A post-heating unit 7 of the machine 200 is operatively active on the mixture to keep its temperature above the dew point.

The vaporisation device 2 is illustrated in figures 2-3.

According to a first embodiment of the invention, for example illustrated in figure 2, the vaporisation device 2 has a first inlet 3a for air and a second inlet 3b for supplying a watery composition containing a liquid sterilising agent. In particular, the liquid sterilising agent is hydrogen peroxide. Preferably, the concentration of hydrogen peroxide in the aqueous compound is about 35%.

Means are provided for generating an air flow 4 which communicate with the first inlet 3a of the vaporisation device 2.

For example, the means for generating an air flow 4 comprise a side-channel blower. Alternatively, for low flow rates, the means for generating an air flow 4 comprise a source of compressed air.

The vaporisation device 2 comprises a heat exchanger 2a, for example, an electrical or vapour heat exchanger of the plate or tube-in-tube type. The heat exchanger 2a is used to provide the necessary heat for changing the phase of the hydrogen peroxide from the liquid to the vapour state. Preferably, the air is filtered, for example, by making it pass through HEPA filters (not illustrated) before being sent to the heat exchanger 2a.

Supply means 5 of the watery composition are placed in communication with the second inlet 3b and configured to supply the watery composition containing the liquid sterilising agent into the vaporisation device 2, through the second inlet 3b.

Preferably, the supply means comprise a nebuliser nozzle 5 arranged along the first air inlet 3a. For example, the nebuliser nozzle 5 is a compressed air nozzle or a piezoelectric nozzle or is a pneumatic nozzle. The watery composition is sent to the nebuliser nozzle 5 through, for example, a pump or a tank pressurised with a gas.

The vaporisation device 2 further comprises an outlet 6 dispensing a mixture comprising air, water vapour and the evaporated sterilising agent. In fact, inside the heat exchanger 2a, the watery composition containing the sterilising agent evaporates and therefore generates the aforementioned mixture of air, water vapour and evaporated sterilising agent which exits from the vaporisation device 2 through the outlet 6.

The evaporated sterilising agent exiting from the heat exchanger 2a has the concentration envisaged for the sterilisation.

Before being sent to the container 100 to be sterilised, the mixture thus obtained crosses the post-heating unit 7 which keeps its temperature above the dew point and therefore keeps it in the gaseous state. The post-heating unit 7 consists of a heat exchanger, for example, powered by electricity or vapour.

Such post-heating unit 7 is placed immediately upstream of a treatment chamber 8 of the container 100 to be sterilised.

In the embodiments described and illustrated herein, the treatment chamber 8 is the internal volume of the container (e.g. bottle) 100, i.e. the space delimited by the walls and the bottom of the container 100.

In an alternative embodiment (not illustrated), the treatment chamber 8 is a chamber containing the container 100, therefore it is the external walls of the bottle 100 that are treated, and potentially also the internal ones.

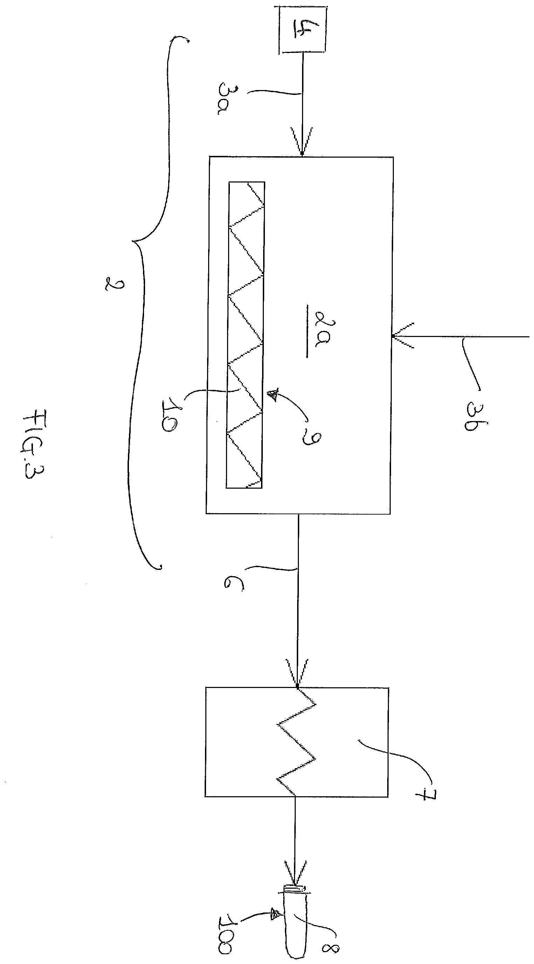

A second possible embodiment of the vaporisation device, for example illustrated in figure 3, differs from the previous one as the vaporisation device 2 comprises a plate 9 associated with electrical resistances 10 so as to be heated. The first inlet 3a and the second inlet 3b are separate and the supply means 5 consist of a conduit configured to make the watery composition drip onto the plate 9. The hydrogen peroxide evaporates and the air is taken away (which acts as a carrier) out of the vaporisation device 2 through the outlet 6. The means for generating the air flow 4 are identical to those described for the first embodiment.

In a further variation (not illustrated), the supply means 5 consist of an ejector that draws the watery composition into the air flow in the vaporisation device 2.

A rotary distributor, not illustrated, is arranged within the isolator 202 and is configured to receive the mixture from the post-heating unit 7 and distribute it to the dispensing nozzles of the treatment stations.

An example of a rotary distributor is described and illustrated in document WO2012/150513.

In particular, the vaporisation device 2 is arranged in a lateral zone of the rotary carousel 201 and the post-heating unit 7 is located close to the rotary carousel 201.

In accordance with a preferred embodiment, the post-heating unit 7 is located on an upper external surface of the isolator 202, upstream of the rotary distributor.

Alternatively, the post-heating unit 7 is located inside the isolator 202, upstream of the rotary distributor.

In other words, the post-heating unit 7 is located in proximity to the machine 200 so as to be able to bring the temperature of the vapours to values higher than the minimum set for obtaining the required sterilisation performance.

The power of the post-heating unit 7 is retroactively regulated as a function of the temperature of the mixture exiting from the post-heating unit 7 measured at the inlet of a machine 200. By regulating and monitoring such temperature it is possible to have a temperature at the nozzles higher than the minimum temperature (e.g. 80°C).

The function of the machine for sterilising containers, according to the present invention, is described below.

The air is supplied into the vaporisation device 2 and the watery composition containing the liquid sterilising agent is introduced into the air flow. Such introduction preferably takes place by injection, for example, by nebulising the watery composition through the nebuliser nozzle 5. See, for example, the first embodiment illustrated in figure 2. Alternatively, the air is supplied into the vaporisation device 2 and the watery composition containing the liquid sterilising agent is made to drip onto the plate 9 (see figure 3).

In the heat exchanger 2a the watery composition containing the sterilising agent evaporates completely. Thus, the mixture of air, water vapour and evaporated sterilising agent is obtained.

The mixture thus obtained passes into the post-heating unit 7 placed immediately upstream of the treatment chamber 8. Advantageously, the post-heating unit 7 allows the mixture to be kept in the gaseous state compensating for losses suffered in the transport conduit of the mixture (not illustrated).

In particular, the vaporisation device 2 is configured to generate a mixture at a temperature of about 110°C and the post-heating unit 7 is configured to increase the temperature of the mixture by about 20°C.

The mixture remains within the post-heating unit 7 for a time interval of about 1 second.

The pressure of the air and/or the mixture is about 300 mbar. Subsequently, the mixture is drawn into the container 100, brushing against its inside walls. In particular, the inside walls of the container 100 are kept at a temperature less than or equal to the condensation temperature of the hydrogen peroxide vapour, i.e. less than 60°C.

In this way, the hydrogen peroxide condenses on the walls. Subsequently, it is evaporated by means of the delivery of hot air.

The concentration of sterilising agent in the mixture is determined by measuring the flow rate of the carrier and the flow rate of sterilising agent at the inlet to the vaporisation device 2, a concentration which is kept constant through a retro-activated control that operates on the dedicated valves so as to keep the flow rates within a pre-established range. According to another variant, the VHP could even remain in the vapour phase (without condensing).

The characteristics of the machine for sterilising containers, according to the present invention, are clear, as are the advantages.

In particular, by heating the gaseous mixture (formed by air, water vapour and hydrogen peroxide) prior to sending it to the container, the concentration of the sterilising agent is kept under control (preventing its condensation prior to the contact with the walls of the container), thus ensuring that the desired degree of sterility is obtained and maintained over time.

This result was obtained by inserting a post-heating unit structurally and functionally separate from the vaporisation device and operating on the mixture containing the completely vaporised sterilising agent in the vaporisation device.

The proposed solution therefore provides for the insertion of a second heat source placed downstream of the vaporisation device, in proximity to the sterilising machine, i.e. to the point in which the hydrogen peroxide vapours are used for the sterilisation.

In this way it is possible to increase the strength and reliability of the system over time.

The specific industrial application of this machine is for the sterilisation of parisons, which will then be blown in a sterile manner within a sterile blower. For this application it is necessary to place the vaporisation device far from the sterilising machine. The post-heating unit is placed in proximity to the sterilising machine, for example, on the roof of its isolator, just before the inlet to the isolator of the pipe which takes the hydrogen peroxide vapours to the rotary distributor. Installation outside the isolator is therefore simple and safe, but it is also possible to install the post-heating unit inside the isolator of the sterilising machine.

## Claims

1. Machine (200) for sterilising containers (100) comprising:
a rotary carousel (201) provided with a plurality of treatment stations of the containers (100), in each of said treatment station being arranged a dispensing nozzle;
an isolator (202) configured for separating the rotary carousel (201) from the external environment;
means for generating an air flow (4);
a vaporisation device (2) configured to generate a mixture of air, water vapour and evaporated sterilising agent, said vaporisation device (2) comprising a first inlet (3a) communicating with said means for generating an air flow (4) so as to feed air into the vaporisation device (2), a second inlet (3b) in communication with supply means (5) configured so as to supply a watery composition containing a liquid sterilising agent inside the vaporisation device (2) and an outlet (6) through which said mixture exits,
wherein it comprises:
a post-heating unit (7) operatively active on said mixture so as to maintain the temperature thereof above a dew point temperature, said post-heating unit (7) being placed immediately upstream of a treatment chamber (8) of the container (100) to be sterilised and being structurally and functionally separate from said vaporisation device (2);
a rotary distributor arranged inside the isolator (202) and configured to receive the mixture from the post-heating unit (7) and to distribute the mixture towards the dispensing nozzles of the treatment stations,
said vaporisation device (2) being arranged in a lateral zone of the rotary carousel (201) and wherein said post-heating unit (7) being located in proximity of the rotary carousel (201).

2. Machine (200) for sterilising containers (100) according to claim 1, wherein said post-heating unit (7) is located on an upper external surface of the isolator (202) upstream of the rotary distributor.

3. Machine (200) for sterilising containers (100) according to claim 1, wherein said post-heating unit (7) is located on a roof of said isolator (202).

4. Machine (200) for sterilising containers (100) according to claim 1, wherein said post-heating unit (7) is located inside the isolator (202), upstream of the rotary distributor.

5. Machine (200) for sterilising containers (100) according to any of the preceding claims, wherein said post-heating unit (7) consists of a heat exchanger.

6. Machine (200) for sterilising containers (100) according to any of the preceding claims, wherein said vaporisation device (2) comprises a heat exchanger (2a).

7. Machine (200) for sterilising containers (100) according to claim 1, wherein said supply means (5) comprise a nebulising nozzle (5) arranged along said first inlet (3a).

8. Machine (200) for sterilising containers (100) according to claim 7, wherein said nebulising device (5) is selected from among: a pneumatic nozzle, a piezoelectric nozzle or a compressed air nozzle.

9. Machine (200) for sterilising containers (100) according to claim 1 or 7 or 8, wherein said vaporisation device (2) comprises a plate (9) associated to electrical resistances (10) so as to be heated in use, and wherein the supply means (5) comprise a conduit configured to drip the watery composition on the plate (9).

## Patentansprüche

1. Maschine (200) zum Sterilisieren von Behältern (100), umfassend:
ein Drehkarussell (201), das mit einer Vielzahl von Behandlungsstationen der Behälter (100) versehen ist, wobei in einer jeden der Behandlungsstationen eine Ausgabedüse angeordnet ist;
einen Isolator (202), der zum Trennen des Drehkarussells (201) von der äußeren Umgebung ausgelegt ist;
Mittel zum Erzeugen eines Luftstroms (4);
eine Verdampfungsvorrichtung (2), die dazu ausgelegt ist, ein Gemisch aus Luft, Wasserdampf und verdampftem Sterilisationsmittel zu erzeugen, wobei die Verdampfungsvorrichtung (2) einen ersten Einlass (3a), der mit den Mitteln zum Erzeugen eines Luftstroms (4) kommuniziert, um Luft in die Verdampfungsvorrichtung (2) zuzuführen, einen zweiten Einlass (3b), der mit Zuführmitteln (5) kommuniziert, die ausgelegt sind, um eine wässrige Zusammensetzung, die ein flüssiges Sterilisationsmittel enthält, innerhalb der Verdampfungsvorrichtung (2) zuzuführen, und einen Auslass (6), durch den das Gemisch austritt, umfasst,
wobei sie Folgendes umfasst:
eine Nachheizeinheit (7), die betriebswirksam auf das Gemisch wirkt, um dessen Temperatur über einer Taupunkttemperatur zu halten, wobei die Nachheizeinheit (7) unmittelbar vor einer Behandlungskammer (8) des zu sterilisierenden Behälters (100) angeordnet ist und strukturell und funktionell von der Verdampfungsvorrichtung (2) getrennt ist;
einen Drehverteiler, der innerhalb des Isolators (202) angeordnet und ausgelegt ist, um das Gemisch von der Nachheizeinheit (7) aufzunehmen und das Gemisch in Richtung der Ausgabedüsen der Behandlungsstationen zu verteilen,
wobei die Verdampfungsvorrichtung (2) in einer seitlichen Zone des Drehkarussells (201) angeordnet ist und wobei die Nachheizeinheit (7) sich in der Nähe des Drehkarussells (201) befindet.

2. Maschine (200) zum Sterilisieren von Behältern (100) nach Anspruch 1, wobei sich die Nachheizeinheit (7) an einer oberen Außenfläche des Isolators (202) stromaufwärts des Drehverteilers befindet.

3. Maschine (200) zum Sterilisieren von Behältern (100) nach Anspruch 1, wobei sich die Nachheizeinheit (7) auf einem Dach des Isolators (202) befindet.

4. Maschine (200) zum Sterilisieren von Behältern (100) nach Anspruch 1, wobei sich die Nachheizeinheit (7) innerhalb des Isolators (202) stromaufwärts des Drehverteilers befindet.

5. Maschine (200) zum Sterilisieren von Behältern (100) nach einem der vorhergehenden Ansprüche, wobei die Nachheizeinheit (7) aus einem Wärmetauscher besteht.

6. Maschine (200) zum Sterilisieren von Behältern (100) nach einem der vorhergehenden Ansprüche, wobei die Verdampfungsvorrichtung (2) einen Wärmetauscher (2a) umfasst.

7. Maschine (200) zum Sterilisieren von Behältern (100) nach Anspruch 1, wobei die Zuführmittel (5) eine Vernebelungsdüse (5) umfassen, die entlang des ersten Einlasses (3a) angeordnet ist.

8. Maschine (200) zum Sterilisieren von Behältern (100) nach Anspruch 7, wobei die Vernebelungsvorrichtung (5) ausgewählt ist aus: einer pneumatischen Düse, einer piezoelektrischen Düse oder einer Druckluftdüse.

9. Maschine (200) zum Sterilisieren von Behältern (100) nach Anspruch 1 oder 7 oder 8, wobei die Verdampfungsvorrichtung (2) eine Platte (9) umfasst, die mit elektrischen Widerständen (10) assoziiert ist, um im Gebrauch erhitzt zu werden, und wobei die Zuführmittel (5) eine Leitung umfassen, die ausgelegt ist, um die wässrige Zusammensetzung auf die Platte (9) zu tropfen.

## Revendications

1. Machine (200) de stérilisation de récipients (100), comprenant :
un carrousel rotatif (201) pourvu d'une pluralité de postes de traitement des récipients (100), dans chacun desdits postes de traitement étant disposée une buse de distribution ;
un isolateur (202) configuré pour séparer le carrousel rotatif (201) de l'environnement extérieur ;
des moyens de génération d'un flux d'air (4) ;
un dispositif de vaporisation (2) configuré pour générer un mélange d'air,
de vapeur d'eau et d'agent stérilisant évaporé, ledit dispositif de vaporisation (2) comprenant une première entrée (3a) communiquant avec lesdits moyens de génération d'un flux d'air (4) afin d'alimenter en air le dispositif de vaporisation (2), une seconde entrée (3b) en communication avec des moyens d'alimentation (5) configurés pour alimenter une composition aqueuse contenant un agent stérilisant liquide à l'intérieur du dispositif de vaporisation (2) et une sortie (6) à travers laquelle ledit mélange sort,
dans laquelle elle comprend :
une unité de postchauffage (7) fonctionnellement active sur ledit mélange de sorte à maintenir sa température au-dessus d'une température de point de rosée, ladite unité de postchauffage (7) étant placée immédiatement en amont d'une chambre de traitement (8) du récipient (100) à stériliser et étant structurellement et fonctionnellement séparée dudit dispositif de vaporisation (2) ;
un distributeur rotatif disposé à l'intérieur de l'isolateur (202) et configuré pour recevoir le mélange provenant de l'unité de postchauffage (7) et pour distribuer le mélange vers les buses de distribution des postes de traitement,
ledit dispositif de vaporisation (2) étant disposé dans une zone latérale du carrousel rotatif (201) et dans laquelle ladite unité de postchauffage (7) est située à proximité du carrousel rotatif (201).

2. Machine (200) de stérilisation de récipients (100) selon la revendication 1, dans laquelle ladite unité de postchauffage (7) est située sur une surface externe supérieure de l'isolateur (202) en amont du distributeur rotatif.

3. Machine (200) de stérilisation de récipients (100) selon la revendication 1, dans laquelle ladite unité de postchauffage (7) est située sur un toit dudit isolateur (202).

4. Machine (200) de stérilisation de récipients (100) selon la revendication 1, dans laquelle ladite unité de postchauffage (7) est située à l'intérieur de l'isolateur (202), en amont du distributeur rotatif.

5. Machine (200) de stérilisation de récipients (100) selon l'une quelconque des revendications précédentes, dans laquelle ladite unité de postchauffage (7) consiste en un échangeur de chaleur.

6. Machine (200) de stérilisation de récipients (100) selon l'une quelconque des revendications précédentes, dans laquelle ledit dispositif de vaporisation (2) comprend un échangeur de chaleur (2a).

7. Machine (200) de stérilisation de récipients (100) selon la revendication 1, dans laquelle lesdits moyens d'alimentation (5) comprennent une buse de nébulisation (5) disposée le long de ladite première entrée (3a).

8. Machine (200) de stérilisation de récipients (100) selon la revendication 7, dans laquelle ledit dispositif de nébulisation (5) est choisi parmi : une buse pneumatique, une buse piézoélectrique ou une buse à air comprimé.

9. Machine (200) de stérilisation de récipients (100) selon la revendication 1 ou 7 ou 8, dans laquelle ledit dispositif de vaporisation (2) comprend une plaque (9) associée à des résistances électriques (10) de manière à être chauffée en fonctionnement, et dans laquelle les moyens d'alimentation (5) comprennent un conduit configuré pour faire tomber goutte à goutte la composition aqueuse sur la plaque (9).
